# EUROPEAN PATENT APPLICATION

(11) **EP 4 541 387 A1**
(43) Date of publication of application: **23.04.2025**
(21) Application number: 23306804.8
(22) Date of filing: 16.10.2023
(51) Int. Cl.: A61M 5/14, A61M 5/315, A61M 5/145, A61M 5/20, A61M 5/178

(54) **SEQUENTIAL-INJECTION SYRINGE**

(71) Applicant: Becton Dickinson France, 38800 Le Pont-de-Claix (FR)
(72) Inventor: LEHEE, Guillaume, 38340 Voreppe (FR); BRUNET, Claire, 38000 Grenoble (FR)
(74) Representative: Germain Maureau

(57) **Abstract**

Provided herein is a sequential-injection syringe having a barrel with a proximal end, a distal end having an opening, and a sidewall therebetween defining a syringe interior, a plunger rod at least partially received within the syringe interior and slidable relative to the barrel, the plunger rod having a proximal end and a distal end, a plunger stopper arranged at the distal end of the plunger rod, a valve stopper arranged in the syringe interior and configured to separate the syringe interior into a first chamber and a second chamber, a concentrated pharmaceutical composition received within the second chamber, and a diluent received within the first chamber.

## Description

### BACKGROUND OF THE INVENTION

### Field of the Invention

Provided herein are sequential-injection syringes for delivery of compositions.

### Description of Related Art

A number of therapeutic compositions exhibit increased stability when the concentration of the active pharmaceutical ingredient (API) is present in higher levels in the composition. However, increasing the concentration of the API results in a lessened volume of therapeutic composition, and thus any dead volume within the container holding the therapeutic composition becomes a greater issue. Accordingly, there is a need in the art for drug delivery devices that effectively deal with dead volume and allow for an entire dose of therapeutic composition to be delivered to patients.

### SUMMARY OF THE INVENTION

Provided herein is a sequential-injection syringe having a barrel with a proximal end, a distal end having an opening, and a sidewall therebetween defining a syringe interior, a plunger rod at least partially received within the syringe interior and slidable relative to the barrel, the plunger rod having a proximal end and a distal end, a plunger stopper arranged at the distal end of the plunger rod, a valve stopper arranged in the syringe interior and configured to separate the syringe interior into a first chamber and a second chamber, a concentrated pharmaceutical composition received within at least one of the first chamber and the second chamber, and a diluent received within at least one of the other of the first chamber and the second chamber.

Further non-limiting embodiments are set forth in the following numbered clauses:

Clause 1. A sequential-injection syringe, comprising: a barrel comprising a proximal end, a distal end having an opening, and a sidewall therebetween defining a syringe interior; a plunger rod at least partially received within the syringe interior and slidable relative to the barrel, the plunger rod having a proximal end and a distal end; a plunger stopper arranged at the distal end of the plunger rod; a valve stopper arranged in the syringe interior and configured to separate the syringe interior into a first chamber and a second chamber; a concentrated pharmaceutical composition received within at least one of the first chamber and the second chamber; and a diluent received within at least one of the other of the first chamber and the second chamber.

Clause 2. The sequential-injection syringe of clause 1, wherein the concentrated pharmaceutical composition is received within the second chamber and the diluent is received within the first chamber.

Clause 3. The sequential-injection syringe of clause 1, wherein the concentrated pharmaceutical composition is received within the first chamber and the diluent is received within the second chamber.

Clause 4. The sequential-injection syringe of any of clauses 1-3, wherein a concentration of the pharmaceutical composition received within the second chamber is at least 10 µg/ml, optionally at least 100 µg/m.

Clause 5. The sequential-injection syringe of any of clauses 1-4, wherein the pharmaceutical composition comprises mRNA.

Clause 6. The sequential-injection syringe of any of clauses 1-5, wherein the diluent is water for injection or a buffer.

Clause 7. The sequential-injection syringe of any of clauses 1-6, wherein the barrel comprises glass or plastic.

Clause 8. The sequential-injection syringe of any of clauses 1-7, wherein an inner surface of the barrel is coated with a friction-reducing composition.

Clause 9. The sequential-injection syringe of any of clauses 1-8, wherein the valve stopper and/or the plunger stopper comprise an elastomeric material.

Clause 10. The sequential-injection syringe of any of clauses 1-9, wherein the valve stopper and/or the plunger stopper are coated with a friction-reducing material.

Clause 11. The sequential-injection syringe of any of clauses 1-10, wherein the valve stopper is configured to move simultaneously co-extensively with the plunger rod, such that a ratio of a volume of the first chamber to a volume of the second chamber remains substantially constant during an initial phase of plunger rod movement.

Clause 12. The sequential-injection syringe of any of clauses 1-11, wherein the valve stopper is configured such that the second chamber is not in fluid communication with the first chamber during the initial phase of plunger rod movement.

Clause 13. The sequential-injection syringe of any of clauses 1-12, wherein as the plunger rod reaches its distal-most position, a hydrostatic pressure disequilibrium within the syringe interior causes the second chamber to be in fluid communication with the first chamber.

Clause 14. The sequential-injection syringe of any of clauses 1-13, wherein the valve stopper comprises one or more reversibly-sealable openings therethrough.

Clause 15. The sequential-injection syringe of any of clauses 1-14, wherein causing the second chamber to be in fluid communication with the first chamber results in flushing of a dead volume of the first chamber through the opening.

Clause 16. The sequential-injection syringe of any of clauses 1-15, wherein the dead volume is between about 2.5 µl and about 70 µl.

Clause 17. A kit comprising one or more sequential-injection syringes of any of clauses 1-16.

Clause 18. A sequential-injection syringe, including a barrel comprising a proximal end, a distal end having an opening, and a sidewall therebetween defining a syringe interior; a plunger rod at least partially received within the syringe interior and slidable relative to the barrel, the plunger rod having a proximal end and a distal end; a plunger stopper arranged at the distal end of the plunger rod; a valve stopper arranged in the syringe interior and configured to separate the syringe interior into a first chamber and a second chamber; a concentrated pharmaceutical composition received within at least one of the first chamber and the second chamber; and a second pharmaceutical composition received within at least one of the other of the first chamber and the second chamber, the second pharmaceutical composition different than the concentrated pharmaceutical composition.

Clause 19. The sequential-injection syringe of clause 18, wherein the concentrated pharmaceutical composition is received within the second chamber and the second pharmaceutical composition is received within the first chamber.

Clause 20. The sequential-injection syringe of clause 18, wherein the concentrated pharmaceutical composition is received within the first chamber and the second pharmaceutical composition is received within the second chamber.

### BRIEF DESCRIPTION OF THE DRAWINGS

**FIG. 1** is a cross-sectional view of a syringe according to non-limiting embodiments described herein;
**FIG. 2** is a cross-sectional view of a syringe according to non-limiting embodiments described herein; and
**FIG. 3** is a cross-sectional view of a syringe according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

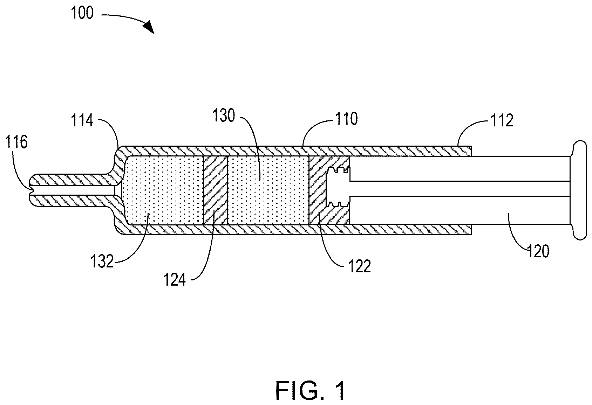

With reference to FIG. 1, shown is a non-limiting embodiment of a sequential-injection syringe 100. Sequential-injection syringe 100 may be a pre-filled syringe, having a barrel 110 with a proximal end 112 and distal end 114 and defining an interior adapted or configured to hold one or a plurality of therapeutic compositions. Barrel 110 can be formed of any suitable material. In non-limiting embodiments or aspects, barrel 110 is formed of glass or a polymer, such as a plastic. Distal end 114 of syringe 100 may include an opening 116, at which a needle may be arranged. While not shown, syringe 100 may include one or more safety features relating to such a needle, for example a cap assembly and/or a rigid needle shield (RNS). RNSs are known in the art, and include an inner, elastomeric portion that encases a needle, and an outer, rigid plastic portion.

With continuing reference to FIG. 1, syringe 100 may include a plunger rod 120. Plunger rod 120 is arranged at proximal end of barrel 110, and is slidable relative thereto from a proximal-most position to a distal-most position. Plunger rod 120 may include an elastomeric stopper 122 at a distal end thereof. Syringe 100 may also include a valve stopper 124 received within interior. Valve stopper 124 may be configured such that interior of syringe 100 is divided into two chambers, a first chamber 130 arranged between plunger stopper 122 and valve stopper 124, and a second chamber 132 arranged between valve stopper 124 and distal end 114 of syringe 100. In non-limiting embodiments, valve stopper 124 may be configured such that under a first condition, such as up to a predetermined pressure differential between first chamber 130 and second chamber 132, valve stopper is in a first configuration and maintains fluid isolation between the chambers. In non-limiting embodiments, under a second condition, for example a hydrolytic pressure disequilibrium between the two chambers, valve stopper 124 may assume a second configuration, and place first chamber 130 in fluid communication with second chamber 132. Suitable valve stoppers are disclosed in U.S. Patent Application Publication No. 2022/0134003, the content of which is incorporated herein by reference in its entirety.

Stoppers, and elastomeric materials for use in the same, are known to those of skill in the art. In non-limiting embodiments or aspects, a useful stopper is formed of an elastomeric material, such as, but not limited to, natural or synthetic rubber, thermoset elastomers, thermoplastic elastomers, and liquid silicone rubber (LSR). In non-limiting embodiments or aspects, the rubber is butyl rubber (IIR), isoprene rubber (IR), butadiene rubber (BR), styrene-butadiene rubber (SBR), ethylene-propylene rubber (EPM), ethylene-propylene-diene rubber (EPDM), chlorosulphonated polyethylene (CSM), ethylene-vinyl acetate copolymer (EVA), styrene-isoprene rubber (SIR), thermoplastic elastomers, and/or natural rubbers.

In non-limiting embodiments or aspects, the stopper can be formed of an elastomeric copolymer, including, without limitation, thermoplastic elastomers, thermoplastic vulcanizates, styrene copolymers such as styrene-butadiene (SBR or SBS) copolymers, styrene-isoprene (SIS) block polymers, or styrene-isoprene/butadiene (SIBS), in which the content of styrene in the styrene block copolymer ranges from about 10% to about 70%, and preferably from about 20% to about 50%. The elastomeric composition can include, without limitation, antioxidants and/or inorganic reinforcing agents to preserve the stability of the elastomer composition, a vulcanizing agent, a vulcanizing accelerator, a vulcanizing activator, a processing aid, a filler, etc., to maintain and improve the physical properties and heat resistance of the rubber material.

In non-limiting embodiments or aspects, plunger stopper 122, valve stopper 124, and/or inner facing surface of barrel 110 sidewall can include a coating thereon. Coatings, for example those for decreasing friction between a stopper and sidewall and/or for reducing interactions between the elastomeric material of the stopper and the medicament, for example siloxane-based coatings, are known to those of skill in the art, and are described in, for example, U.S. Patent Application Publication Nos. 2014/0221934 and 2014/0110297, the contents of which are incorporated herein by reference in their entirety. In one non-limiting aspect, the face of the valve and/or plunger stopper can be coated with a material to limit substances leaching and being extracted from the valve or plunger stopper into the concentrated pharmaceutical composition, diluent, and/or second pharmaceutical composition.

With continuing reference to FIG. 1, shown is syringe 100 in a pre-use configuration. In such a configuration, plunger rod 120 is in a proximal position. In non-limiting embodiments, first chamber 130 may have a first volume, and second chamber 132 may have a second volume. In non-limiting embodiments, the first volume and the second volume may be in a predetermined ratio. In a static condition, valve stopper 124 in first configuration maintains fluid isolation between first chamber 130 and second chamber 132. In non-limiting embodiments, a therapeutic composition, including an active pharmaceutical ingredient (API), is received in the second chamber 132, and a diluent is received within first chamber 130. In other non-limiting embodiments, a therapeutic composition, including an active pharmaceutical ingredient (API), is received in the first chamber 130, and a diluent is received within second chamber 132. In still other configurations, a second pharmaceutical composition, including a different API, may replace and/or supplement the diluent in either the first chamber 130 or the second chamber 132. The API may be any therapeutic. In non-limiting embodiments the therapeutic is mRNA-based. In non-limiting embodiments, the API is included in the therapeutic composition at a concentration of at least about 1 µg/ml, at least about 10 µg/ml, at least about 25 µg/ml, at least about 70 µg/ml, and/or at least about 100 µg/ml, all values and subranges therebetween inclusive. With regard to the first chamber 130, suitable diluents are known to those of skill in the art, and may include water-for-injection (WFI), saline, buffers, and/or other composition known to those of skill in the art for being safe for administration to a patient. In non-limiting embodiments, the diluent is matched to the therapeutic composition.

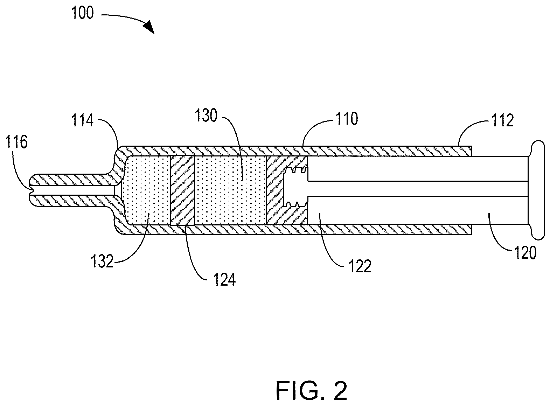

With reference to FIG. 2, shown is a use configuration of syringe 100. Plunger rod 120 is shown along a path of travel from a proximal positon (FIG. 1) as therapeutic composition is delivered through opening 116 to a patient. In non-limiting embodiments, because a pressure difference between first chamber 130 and second chamber 132 does not exceed a pre-determined threshold to which valve stopper is configured, valve stopper 124 remains in a first configuration and maintains fluid isolation of first chamber 130 from second chamber 132. In non-limiting embodiments, plunger stopper 122 and/or valve stopper 124 are configured such that valve stopper moves distally at substantially the same rate as plunger stopper 122. In non-limiting embodiments, for example based on the substantially similar movement of plunger stopper 122 and valve stopper 124, the predetermined ratio of first volume and second volume is maintained during at least an initial period of drug delivery.

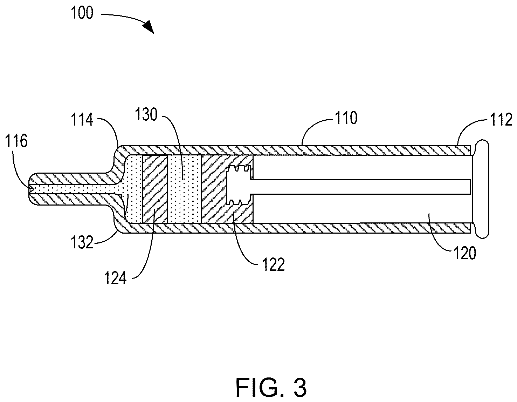

Turning to FIG. 3, shown is a use configuration of syringe 100 following the use configuration of FIG. 2, and in which plunger rod 120 has been advanced distally to a position in which therapeutic composition within second chamber 132 has been delivered to a patient. In non-limiting embodiments, only a dead volume of the therapeutic composition remains in second chamber 132. In non-limiting embodiments, as valve stopper 124 has advanced as far distally within barrel 110 as possible, pressure buildup within first chamber 130 results in hydrolytic pressure disequilibrium, and valve stopper 124 switches to a second configuration, placing first chamber 130 in fluid communication with second chamber 132. In such a second configuration, diluent in first chamber 130 may flush the dead volume of therapeutic composition remaining within second chamber 132, thereby delivering any remaining volume of therapeutic composition to the patient.

Although the invention has been described in detail for the purpose of illustration based on what are currently considered to be the most practical and preferred embodiments, it is to be understood that such detail is solely for that purpose and that the invention is not limited to the disclosed embodiments, but on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A sequential-injection syringe, comprising:
a barrel comprising a proximal end, a distal end having an opening, and a sidewall therebetween defining a syringe interior;
a plunger rod at least partially received within the syringe interior and slidable relative to the barrel, the plunger rod having a proximal end and a distal end;
a plunger stopper arranged at the distal end of the plunger rod;
a valve stopper arranged in the syringe interior and configured to separate the syringe interior into a first chamber and a second chamber;
a concentrated pharmaceutical composition received within at least one of the first chamber and the second chamber; and
a diluent received within at least one of the other of the first chamber and the second chamber.

2. The sequential-injection syringe of claim 1, wherein the concentrated pharmaceutical composition is received within the second chamber and the diluent is received within the first chamber.

3. The sequential-injection syringe of claim 1, wherein the concentrated pharmaceutical composition is received within the first chamber and the diluent is received within the second chamber.

4. The sequential-injection syringe of any of claims 1-3, wherein a concentration of the pharmaceutical composition received within the second chamber is at least 10 µg/ml, optionally at least 100 µg/ml.

5. The sequential-injection syringe of any of claims 1-4, wherein the pharmaceutical composition comprises mRNA.

6. The sequential-injection syringe of any of claims 1-5, wherein the diluent is water for injection or a buffer.

7. The sequential-injection syringe of any of claims 1-6, wherein the barrel comprises glass or plastic.

8. The sequential-injection syringe of any of claims 1-7, wherein an inner surface of the barrel is coated with a friction-reducing composition.

9. The sequential-injection syringe of any of claims 1-8, wherein the valve stopper and/or the plunger stopper comprise an elastomeric material.

10. The sequential-injection syringe of any of claims 1-9, wherein the valve stopper and/or the plunger stopper are coated with a friction-reducing material.

11. The sequential-injection syringe of any of claims 1-10, wherein the valve stopper is configured to move simultaneously co-extensively with the plunger rod, such that a ratio of a volume of the first chamber to a volume of the second chamber remains substantially constant during an initial phase of plunger rod movement.

12. The sequential-injection syringe of any of claims 1-11, wherein the valve stopper is configured such that the second chamber is not in fluid communication with the first chamber during the initial phase of plunger rod movement.

13. The sequential-injection syringe of any of claims 1-12, wherein as the plunger rod reaches its distal-most position, a hydrostatic pressure disequilibrium within the syringe interior causes the second chamber to be in fluid communication with the first chamber.

14. The sequential-injection syringe of any of claims 1-13, wherein the valve stopper comprises one or more reversibly-sealable openings therethrough.

15. The sequential-injection syringe of any of claims 1-14, wherein causing the second chamber to be in fluid communication with the first chamber results in flushing of a dead volume of the first chamber through the opening.

16. The sequential-injection syringe of any of claims 1-15, wherein the dead volume is between about 2.5 µl and about 70 µl.

17. A kit comprising one or more sequential-inj ection syringes of any of claims 1-16.

18. A sequential-injection syringe, comprising:
a barrel comprising a proximal end, a distal end having an opening, and a sidewall therebetween defining a syringe interior;
a plunger rod at least partially received within the syringe interior and slidable relative to the barrel, the plunger rod having a proximal end and a distal end;
a plunger stopper arranged at the distal end of the plunger rod;
a valve stopper arranged in the syringe interior and configured to separate the syringe interior into a first chamber and a second chamber;
a concentrated pharmaceutical composition received within at least one of the first chamber and the second chamber; and
a second pharmaceutical composition received within at least one of the other of the first chamber and the second chamber, the second pharmaceutical composition different than the concentrated pharmaceutical composition.

19. The sequential-injection syringe of claim 18, wherein the concentrated pharmaceutical composition is received within the second chamber and the second pharmaceutical composition is received within the first chamber.

20. The sequential-injection syringe of claim 18, wherein the concentrated pharmaceutical composition is received within the first chamber and the second pharmaceutical composition is received within the second chamber.
